# EUROPEAN PATENT SPECIFICATION

(11) **EP 4 349 291 B1**
(45) Date of publication and mention of the grant of the patent: **04.06.2025**
(21) Application number: 23201108.0
(22) Date of filing: 02.10.2023
(51) Int. Cl.: A61B 18/14, A61B 34/00

(54) **SEAMLESS SWITCHING BETWEEN DIFFERENT MODES OF TISSUE ABLATION**
NAHTLOSES UMSCHALTEN ZWISCHEN VERSCHIEDENEN GEWEBEABLATIONSMODI
COMMUTATION SANS COUPURE ENTRE DIFFÉRENTS MODES D'ABLATION DE TISSU

(30) Priority: 03.10.2022 US 202217958760
(43) Date of publication of application: 10.04.2024
(73) Proprietor: BIOSENSE WEBSTER (ISRAEL) LTD., 2066717 Yokneam (IL)
(72) Inventor: GOVARI, Assaf, 2066717 Yokneam (IL); ALTMANN, Andres Claudio, 2066717 Yokneam (IL)
(74) Representative: Carpmaels & Ransford LLP

(56) References cited:
- WO-A1-2021/102230
- US-A1- 2008 004 534
- US-A1- 2014 330 266
- US-A1- 2021 401 491
- US-A1- 2022 061 912

## Description

### FIELD OF THE DISCLOSURE

The present disclosure relates generally to medical devices, and particularly to methods and systems for seamless switching between different ablation modes of tissue.

### BACKGROUND OF THE DISCLOSURE

Tissue ablation is used in various medical procedures, such as in treating arrhythmias in a patient heart by a physician using one or more ablation catheters for generating a lesion at a predefined site within the patient heart. There are different ablation modes, and sometimes during the ablation, the physician may decide to switch from a first ablation mode to a second ablation mode, which is more suitable for obtain the desired lesion.

US 2022/061912 A1 teaches a medical apparatus that includes a probe configured for insertion into a body of a patient and comprising a plurality of electrodes configured to contact tissue within the body. The medical apparatus further includes an electrical signal generator configured to apply between one or more pairs of the electrodes sinusoidal radio-frequency (RF) signals of first and second types in alternation. The signals of the first type have a first voltage sufficient to cause irreversible electrophoresis (IRE) in the tissue contacted by the electrodes and a first power that is insufficient to thermally ablate the tissue, and the signals of the second type have a second power sufficient to thermally ablate the tissue contacted by the electrodes and a second voltage that is insufficient to cause IRE in the tissue.

### SUMMARY OF THE INVENTION

The invention is defined in claim 1. Embodiments of the invention are defined in the dependent claims. Any methods of treatment described hereinafter are presented for illustrative purposes only and do not, by themselves, form part of the invention.

The present disclosure will be more fully understood from the following detailed description of the examples thereof, taken together with the drawings in which:

### BRIEF DESCRIPTION OF THE DRAWINGS

Fig. 1 is a schematic, pictorial illustration of a catheter-based electrophysiology mapping and ablation system, in accordance with an example of the present disclosure;
Fig. 2 is a schematic, pictorial illustration of annotations that are indicative of PFA energy and RF energy applied to the tissue of heart, and are superimposed on anatomical map of the heart, in accordance with an example of the present disclosure; and
Fig. 3 is a flow chart that schematically illustrates a method for seamless switching between PFA-based and RF-based ablation modes, in accordance with an example of the present disclosure.

### DETAILED DESCRIPTION OF EXAMPLES

### OVERVIEW

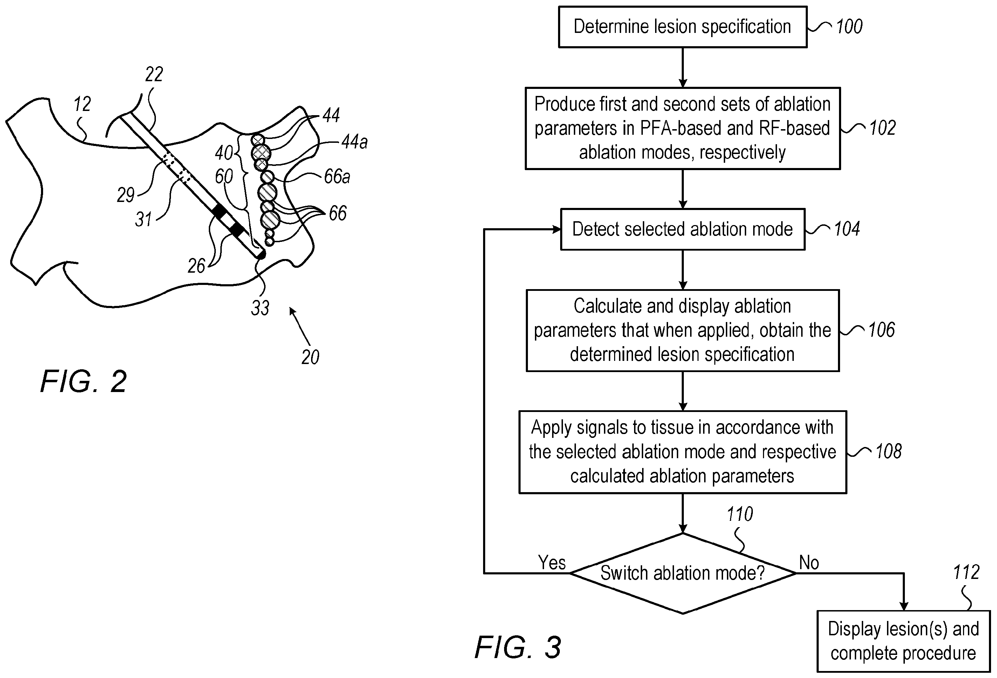

Various ablation modes, such as but not limited to Pulsed Field Ablation (PFA), and radiofrequency (RF) ablation, may be used for treating arrhythmia in a patient heart using one or more catheters having suitable electrodes. PFA may be used for ablating tissue cells by applying to tissue high-voltage pulses. Cellular destruction occurs when the transmembrane potential exceeds a threshold, leading to cell death and irreversible electroporation (IRE) of the tissue, resulting in the formation of a lesion in a respective section of the heart. In PFA-based ablation procedures, a sequence of microsecond high-voltage electrical pulses is applied to the tissue intended to be ablated, and in RF-based ablation, a lesion is formed by applying to the tissue electrical pulses having a lower electrical power and a longer duration compared to that of PFA, an example of the different ablation parameters is described in Fig. 2 below.

In some cases, the ablation procedure is carried out along a line, referred to herein as an ablation line, and while applying the ablation pulses along the ablation line, a physician that conducts the ablation may decide to switch between the ablation modes (e.g., from PFA to RF). In such cases, the physician must select a new set of ablation parameters for obtaining the required properties of the lesion. In order to obtain a contiguous lesion having a uniform depth and width along the ablation line, several parameters must be altered. For example, the distance between each pair of ablation points, and contact force between the catheter and the tissue, must be adjusted when switching between PFA-based and RF-based ablation mode. Manual adjustment of the parameters may reduce the quality of the lesion, and may undesirably increase the duration of the ablation procedure.

Examples of the present disclosure that are described hereafter provide techniques for seamless switching between ablation modes by automatically adjusting the suggested ablation parameters in response to switching between ablation modes during a tissue ablation procedure.

In some examples, a system for treating arrhythmia in a patient heart comprises one or more catheters, at least one of the catheters has one or more ablation electrodes configured to apply different types of ablation signals, such as RF ablation signals and PFA ablation signals. The system further comprises a generator configured to generate both types of the ablation signals, and a switching box configured to switch between the PFA-based and RF-based ablation signals applied by the generator.

In some examples, the system comprises a processor, which is configured to receive (e.g., from the physician) a target lesion parameter indicative of a specification of a lesion intended to be formed in the tissue of the heart. For example, the lesion parameters may comprise at least the depth and width of a lesion.

In some examples, the processor is configured to calculate a first set of ablation parameters for the PFA-based ablation mode, and a second set of ablation parameters for the RF-based ablation mode. Alternatively, the processor may select the ablation parameters based on stored data obtained in previous suitable ablation procedures. Note that when the first and second sets of ablation parameters are applied, a lesion having the lesion parameter is expected to be formed in the corresponding location within the patient heart. Examples of lesion parameters and corresponding sets of ablation parameters are described in detail in Fig. 2 below.

In some cases, at a given ablation point along the ablation line, the physician may toggle between the ablation modes while performing the ablation along the ablation line. In some examples, in response to the toggling, the processor is configured to (i) detect which ablation mode (e.g., PFA or RF) has been selected by the physician, (ii) provide guidance for positioning the catheter at an ablation site based on the ablation mode (e.g., PFA or RF) selected, and (iii) apply to the given ablation point ablation signals corresponding to the selected ablation mode.

The disclosed techniques enable seamless switching between ablation modes along the ablation line without preplanning, and therefore, improve the lesion quality and reduce the duration of ablation procedures.

### SYSTEM DESCRIPTION

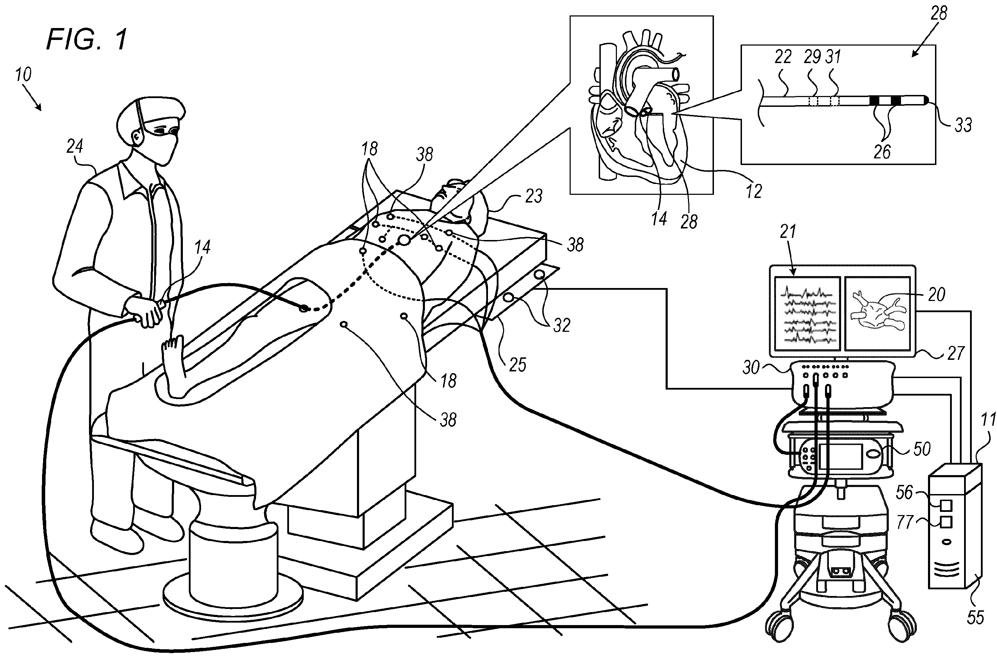

Fig. 1 is a schematic, pictorial illustration of a catheter-based electrophysiology mapping and ablation system 10, in accordance with an example of the present disclosure.

In some examples, system 10 includes multiple catheters, which are percutaneously inserted by a physician 24 through the patient's vascular system into a chamber or vascular structure of a heart 12. Typically, a delivery sheath catheter is inserted into the left or right atrium near a desired location in heart 12. Thereafter, one or more catheters can be inserted into the delivery sheath catheter so as to arrive at the desired location within heart **12.** The plurality of catheters may include catheters dedicated for sensing Intracardiac Electrogram (IEGM) signals, catheters dedicated for ablating and/or catheters adapted to carry out both sensing and ablating. An example catheter 14 that is configured for sensing IEGM is illustrated herein. In some embodiments, physician 24 may place a distal tip 28 of catheter 14 in contact with the heart wall for sensing a target site in heart 12. Additionally, or alternatively, for ablation, physician 24 would similarly place a distal end of an ablation catheter in contact with a target site for ablating tissue intended to be ablated.

In the present example, catheter 14 includes one and preferably multiple electrodes 26 optionally distributed along a shaft 22 at distal tip 28 of catheter **14.** Electrodes 26 and configured to sense the IEGM signals. Catheter 14 may additionally include a position sensor 29 embedded in or near distal tip 28 for tracking position and orientation of distal tip 28. Optionally and preferably, position sensor 29 is a magnetic based position sensor including three magnetic coils for sensing three-dimensional (3D) position and orientation.

In some examples, magnetic based position sensor 29 may be operated together with a location pad 25 including a plurality of (e.g., three) magnetic coils 32 configured to generate a plurality of (e.g., three) magnetic fields in a predefined working volume. Real time position of distal tip 28 of catheter 14 may be tracked based on magnetic fields generated with location pad 25 and sensed by magnetic based position sensor 29. Details of the magnetic based position sensing technology are described, for example, in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

In some examples, catheter 14 includes a contact force sensor 31, which is configured to sense the contact force applied to tissue of heart 12 by distal tip 28, and to produce a signal indicative of the sensed contact force.

In some examples, system 10 includes one or more electrode patches 38 positioned for skin contact on patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. This technique is also referred to herein as Advanced Current Location (ACL) and details of the impedance-based location tracking technology are described in US Patent Nos. 7,536,218; 7,756,576; 7,848,787; 7,869,865; and 8,456,182. In some embodiments, the magnetic based position sensing and the ACL may be applied concurrently, e.g., for improving the position sensing of one or more electrodes coupled to a shaft of a rigid catheter or to flexible arms or splines at the distal tip of another sort of catheter, such as the PentaRay^{®} or OPTRELL^{®} catheters, available from Biosense Webster, Inc., 31A Technology Drive, Irvine, CA 92618.

In some examples, a recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

In some examples, system 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulse trains of pulsed-field ablation (PFA) energy, including monopolar or bipolar high-voltage DC pulses as may be used to effect irreversible electroporation (IRE), or combinations thereof. In the present example, catheter 14 includes an ablation electrode 33 but optionally includes multiple electrodes 33 (not shown), positioned at distal tip 28 and configured to apply the RF energy and/or the pulse trains of PFA energy to tissue of the wall of heart 12.

In some examples, patient interface unit (PIU) 30 is an interface configured to establish electrical communication between catheters, electrophysiological equipment, power supply and a workstation 55 for controlling the operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

In some examples, workstation 55 includes a storage device, a processor 77 with suitable random-access memory, or storage with appropriate operating software stored therein, an interface 56 configured to exchange signals of data (e.g., between processor 77 and another entity of system 10) and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (4) displaying on display device 27 sites of interest such as places where ablation energy has been applied. One commercial product embodying elements of the system 10 is available as the CARTO^{™} 3 System, available from Biosense Webster, Inc., 31A Technology Drive, Irvine, CA 92618.

In some examples, processor 77 receives from contact force sensor 31 a signal indicative of the contact force applied between ablation electrode 33 and the tissue intended to be ablated. Moreover, processor 77 may store one or more contact force thresholds in order to provide physician 24 with an indication of whether the contact force applied between ablation electrode 33 and the tissue intended to be ablated is sufficient for the planned ablation mode (e.g., a first threshold for RF-based ablation mode, and a second different threshold for PFA-based ablation mode).

Fig. 2 is a schematic, pictorial illustration of annotations 44 and 46 that are indicative of PFA energy and RF energy applied to the tissue of heart 12, respectively, and are superimposed over anatomical map 20, in accordance with an example of the present disclosure.

In the example of Fig. 2, a plurality of annotations 44 and an annotation 44a are indicative of the ablation event in response to the PFA energy used for ablating tissue cells of heart 12. Similarly, a plurality of annotations 66 and an annotation 66a are indicative of the lesion formed in response to the RF energy used for ablating tissue cells of heart 12. Note that annotations 44 and 44a are similar and annotations 66 and 66a are similar and the index "a" of annotations 44a and 66a is used herein for the sake of presentation clarity, as will be described below.

In PFA-based ablation, cellular destruction of the tissue occurs when the transmembrane potential exceeds a threshold, which leads to cell death and irreversible electroporation (IRE) of the tissue and results in the formation of a lesion in a section 40 of the wall tissue of heart 12. In PFA-based ablation procedures, a sequence of microsecond high-voltage electrical pulses is applied to the tissue of section 40.

In response to applying RF energy, also referred to herein as RF ablation or RF-based ablation mode, a lesion is formed by applying to the tissue electrical pulses having, compared to that of PFA, a lower electrical power and a longer duration, an example of the different ablation parameters is described below.

Both ablation modes are used to form in the tissue a lesion, but the properties of the lesion may depend on the ablation mode (e.g., PFA-based or RF-based ablation) used for producing them. Moreover, both ablation modes may be carried out by applying the pulses using a unipolar (also referred to herein as monopolar) ablation or using a bipolar ablation (e.g., between two ablation electrodes, in case catheter 14 has two or more ablation electrodes 33). In the present example, the unipolar ablation may be carried out between ablation electrode 33 coupled to shaft 22 of catheter 14 and an indifferent electrode (e.g., one of electrode patches 38). Additionally, or alternatively, system 10 may have any other type of catheter suitable for ablating the tissue and having ablation electrodes, such as but not limited to electrodes 33. Note that the examples of the present disclosure that are described below, are applicable for focal catheter 14 and, *mutatis mutandis,* for other suitable type of catheters, such as a basket catheter, a balloon catheter, a lasso catheter, and other suitable types of catheters having ablation electrodes.

In some cases, the ablation procedure is carried out along sections 40 and 60, also referred to herein as ablation lines, and while applying the ablation energy, physician 24 that conducts the ablation may decide to switch between the ablation modes (e.g., from PFA-based to RF-based). In this example, when moving ablation electrode 33 from section 40 to section 60, physician 24 must select a new set of ablation parameters for obtaining the required properties of the lesion. In order to obtain a contiguous lesion having a uniform depth and width along the ablation line, several parameters must be altered between sections 40 and 60. For example, the voltage and power, pulse duration, number of pulses, and the contact force between distal tip 28 of shaft 22 and the tissue must be adjusted when switching between PFA-based and RF-based ablation modes. It is noted that manual adjustment of these parameters may reduce the quality of the lesion (e.g., due to usage of non-optimal ablation conditions), and may increase the duration of the ablation procedure.

Examples of the present disclosure that are described below provide techniques for automatically adjusting the ablation parameters in response to switching between ablation modes (e.g., between PFA-based and RF-based ablation modes and *vice versa)* during tissue ablation procedure.

In some examples, ablation electrode 33 is configured to apply both RF energy and PFA energy to the tissue of heart 12. In an example configuration, system 10 comprises a PFA pulse generator (PPG) and a RF pulse generator that in the present example are incorporated in a single generator, referred to herein as a generator 50. In another example configuration, system 10 may comprise two separate generators, a PPG for applying the PFA energy and a different generator for applying the RF energy. In both configurations, system 10 may comprise a switching box (not shown) configured to switch between the PFA-based and RF-based ablation modes.

In some examples, display device 27 is configured to receive from physician 24 (e.g., using a graphical user interface displayed thereon) one or more lesion parameters, which are transferred to interface 56 implemented in workstation 55 as described in Fig. 1 above. In the present example, the lesion parameters are indicative of a specification of the lesion intended to be formed in sections 40 and 60 of the tissue of heart 12. For example, the lesion parameters comprise at least one of a lesion depth (e.g., about 5 mm), and a lesion width (e.g., between about 8 mm and 13 mm). In other examples, physician 24 can choose pre-defined types of lesion parameters (e.g., shallow, medium, and deep ablation), and responsively, processor 77 selects ablation parameters that match this level for each of the RF-based and PFA-based ablation modes. Moreover, when using specific types of ablation catheters (e.g., catheters having multiple ablation electrodes, such as electrodes 33, arranged along one or more splines or along a balloon), the lesion parameter may be indicative of the continuity of the lesion.

In some examples, based on the lesion parameters, processor 77 is configured to calculate (i) a first set of ablation parameters for the PFA-based ablation mode, and (ii) a second set of ablation parameters for the RF-based ablation mode. In other examples, processor 77 may have one or more stored lookup tables based on empirical data from previous ablation procedures, and based of the selected lesion parameter, processor 77 selects the first set of ablation parameters for the PFA-based ablation mode, and the second set of ablation parameters for the RF-based ablation mode.

In the example of Fig. 2, the first set of ablation parameters is applied to the tissue along the ablation line of section 40, and the second set of ablation parameters is applied to the tissue along the ablation line of section 60. Note that the first and second sets of ablation parameters are intended to form along sections 40 and 60, respectively, a lesion having the aforementioned lesion parameters defined by physician 24.

For example, physician 24 may define lesion parameters comprising a lesion depth of about 5 mm and a lesion width of about 10 mm along section 60. In this example, processor 77 selects and/or calculates ablation parameters for the RF-based ablation mode, such as power, duration, contact force, temperature, and distance between ablation points. For example, a pulse amplitude of about 35 watts (or any other suitable power between about 20 watts and 50 watts), which is applied to the tissue for about 35 seconds (or for any other suitable time interval between about 10 seconds and 60 seconds).

Similarly, physician 24 may define the same 5 mm depth and 10 mm width along section 40. In this example, processor 77 is configured to calculate ablation parameters for the PFA-based ablation mode, such as but not limited to number of pulses in a train, number of trains, pulse amplitude, and distance between ablation points. For example, a voltage amplitude of about 1500 volts (or any other suitable voltage between about 900 volts and 2000 volts), which is applied to the tissue for about 150 milliseconds (or for any other suitable time interval between about 100 milliseconds and 250 milliseconds). Note that the 150 milliseconds time interval of the PFA energy comprises multiple sequential pulse trains having respective time intervals of about 50 microseconds, each pulse train comprising a sequence of multiple pulses applied to the tissue for a time interval between about 2 microseconds and 5 microseconds. Typically, the distance in RF-based ablation mode is larger compared to that of the PFA-based ablation mode. Processor 77 may display, over display device 27, the distance from the previous ablation point, and lets physician 24 know at what distance to set the next ablation point. When physician 24 switches the ablation mode, processor 77 automatically switches the instruction for where to place the next ablation point. For example, if physician 24 switches from RF-based to PFA-based ablation mode, processor 77 reduces the suggested distance between the previous and the next ablation points. The distance between adjacent ablation points is important for obtaining a contiguous lesion having a uniform depth and width.

Note that after defining the 5 mm depth and 10 mm width of the lesion size, physician 24 can switch at will between RF-based and PFA-based ablation modes, without preplanning, and processor 77 controls generator 50 to apply to electrode 33 the respective set of ablation parameter corresponding to the selected ablation mode and also adjusts the indications provided on the GUI that guides the physician in performing the next ablation. Guidance may include for example guiding the physician in placing the catheter at the next ablation site (at a recommended distance from adjacent ablation site) as well as placing the catheter against the tissue with the recommended contact force.

In some examples, processor 77 is configured to display over display device 27, a toggle switch for toggling between PFA-based and RF-based ablation modes. When conducting the ablation procedure along the ablation lines comprising section 40 and 60, physician 24 may: (i) select the PFA-based ablation mode in the toggle switch, and use catheter 14 for applying to section 40 the PFA energy (also referred to herein as a first ablation mode), and subsequently, (ii) select the RF-based ablation mode in the toggle switch, and use catheter 14 for applying to section 60 the RF energy (also referred to herein as a first ablation mode). Note that physician 24 starts the tissue ablation at the uppermost point of section 40 on the wall tissue of heart 12, and the transition between the first and second ablation modes is carried out at the interface between sections 40 and 60, in the present example, between the locations of annotations 44a and 66a.

In some examples, after concluding the ablation at the point corresponding to annotation 44a and before performing the ablation at the point corresponding to annotation 66a, physician 24 toggles the ablation mode from the PFA-based to the RF-based ablation mode. Note that each of annotations 44, 44a, 66 and 66a is typically displayed over anatomical map 20 after performing the ablation. A GUI indication may be provided to the physician to guide the physician as to where to place the next ablation. Optionally, during PFA shorter distances between ablation sites shown as annotations 66 is recommended as compared to during RF ablation shown as annotations 44. The indicated recommendation may be adjusted based on detecting a switch. Optionally, the recommended position of ablation site, referred to herein as annotation 66a, in relation to ablation site of annotation 44a may be pre-defined and indicated to the physician based on detecting the switch.

In some examples, processor 77 is configured to detect the switched ablation mode (e.g., from PFA-based to RF-based ablation mode), and responsively, to apply the set of parameters of RF energy to the ablation sites of section 60, so as to obtain the predefined lesion parameters (e.g., the lesion depth of about 5 mm and the lesion width of about 10 mm) in section 60. In other words, from the ablation point corresponding to annotation 66a, and along section 60, processor 77 controls catheter 14 to apply to the wall tissue of heart 12 RF energy via catheter 14 and electrode 33.

In some examples, display device 27 is configured to display (i) anatomical map 20, (ii) the lesion parameters (determined by physician 24) and the corresponding ablation parameters (determined by processor 77) for each ablation mode, and (iii) annotations 44 and 66 (e.g., ablation tags) indicative of the ablation event at that location.

Fig. 3 is a flow chart that schematically illustrates a method for seamless switching between PFA-based and RF-based ablation modes, in accordance with an example of the present disclosure.

The method begins at a lesion specification definition step 100, with physician 24 determining the specification of the lesion intended to be formed along sections 40 and 60 of the tissue of heart 12. For example, lesion depth of about 5 mm and lesion width of about 10 mm, as described in detail in Fig. 2 above.

At a preliminary ablation parameter setting step 102, processor 77 selects (e.g., from the aforementioned storage device of workstation 55) first and second sets of predefined (e.g., default) ablation parameters for the PFA-based and the RF-based ablation modes, respectively, as described in Fig. 2 above.

At a detection step 104, processor 77 detects that physician 24 has selected the PFA-based ablation mode in the toggle switch, as described in detail in Fig. 2 above.

At an ablation parameter adjustment setting step 106, processor 77 calculates the ablation parameters (e.g., of the PFA-based ablation mode) that when applied to the tissue, are expected to obtain the lesion specification determined by physician 24 in step 100 above.

At an ablation step 108, processor 77 controls generator 50 and catheter 14 to apply signals of PFA-based energy to the tissue at the ablation points of section 40. Note that the signals of PFA-based energy are selected and adjusted **(e.g.,** calculated) at steps 102 and 106, respectively, and optionally, processor 77 is further configured to display the ablation parameters of the of PFA-based energy on display device 27, so that physician 24 and any other suitable user of system 10 could see the calculated ablation parameters.

At a decision step 110 that in the present example is carried out after concluding the ablation at the position of annotation 44a, physician 24 decided whether s/he wants to switch the ablation mode, **e.g.,** from PFA-based to RF-based, at the position of annotation 66a. Note that the switching between the ablation modes may not be preplanned, and physician 24 may decide to toggle between the ablation modes during the procedure (or alternatively, prior to the procedure). In case physician 24 decides to switch the ablation mode, the method loops back to step 104 and processor 77 (i) detects that physician 24 has toggled from PFA-based to RF-based ablation mode, (ii) calculates the ablation parameters as described in step 106 above, and (iii) controls generator 50 and catheter 14 to apply the RF energy to section 60 of the tissue, based on the ablation parameters calculated in step 106.

At an ablation termination step 112 that concludes the method, after switching to the RF-based ablation mode and concluding the ablation of section 60, processor 77 displays annotations 44 and 66 and controls generator 50 to stop applying the ablation energy to catheter 14.

Alternatively, at decision step 110 physician 24 may decide to continue the ablation by applying PFA energy also to section 60. In this example, processor 77 is configured to retain the ablation parameters of the PFA-based ablation mode, and display annotations 44 and 66 in accordance with the progress of the ablation procedure. Moreover, after concluding the ablation in sections 40 and 60, processor 77 controls generator 50 to stop applying the ablation energy to catheter 14.

Although the examples described herein mainly address electrophysiology procedures applying RF ablation energy and pulse trains of PFA energy to tissue of the heart wall of heart 12, the methods and systems described herein can also be used in other applications, such as in any other switching between different operating modes that include applying signals to any suitable organ of a patient.

It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art.

## Claims

1. A system (10), comprising:
an interface (56), which is configured to receive a lesion parameter indicative of a specification of a lesion intended to be formed in tissue of an organ (12) using at least one of first and second ablation modes intended to be applied sequentially to multiple sections (40, 60) of the tissue; and
a processor (77), which is configured to (i) select, for the first and second ablation modes, first and second sets of parameters, respectively, that when applied to the tissue, form the lesion having the lesion parameter, (ii) detect, for a section selected among the multiple sections (40, 60), whether the first or second ablation mode has been selected, and (iii) control a catheter (22) having at least an ablation electrode (26), to apply to the section ablation signals that are based on the selected ablation mode and are corresponding to the first or second ablation modes.

2. The system according to claim 1, wherein the processor is configured to select the first and second sets of parameters by: (i) calculating the first and second ablation parameters, or (ii) selecting the first and second ablation parameters based on stored empirical data corresponding to the first and second ablation modes.

3. The system according to claim 1 or claim 2, wherein the organ comprises a heart of a patient, and wherein the processor is configured to: (i) select the first set of parameters by selecting pulse trains of pulsed-field ablation (PFA) energy, and (ii) select the second set of parameters by selecting radiofrequency (RF) energy.

4. The system according to claim 3, wherein the processor is configured to control the catheter to apply the selected pulse trains of PFA energy to the section, and subsequently, (i) detect that the second ablation mode has been selected for applying to a subsequent section selected among the multiple sections, second ablation signals that are subsequent to the first ablation signals, and (iii) control the catheter to apply the selected RF energy to the subsequent section.

5. The system according to claim 1 or claim 2, wherein the lesion parameter comprises at least a geometrical parameter selected from a list of geometrical parameters consisting of: (i) a depth of the lesion, (ii) a width of the lesion, and (iii) continuity of the lesion.

6. The system according to claim 5, wherein the interface is configured to receive from a user the lesion parameter determined based on instructions indicative of the geometrical parameters, and the processor is configured to select the first and second sets of parameters based on the instructions determined by the user.

7. The system according to claim 1, wherein the processor is configured to: (i) select first default parameters for selecting the first set, and second default parameters for selecting the second set, and (ii) select the first and second sets of parameters by adjusting the first and second default parameters based on an electro-anatomical mapping of the tissue.

8. The system according to claim 1 or claim 2, and comprising a display, wherein, in response to detecting the selection of the first or second ablation mode, the processor is configured to display to a user the first or second sets of parameters, respectively.

9. The system according to claim 8, wherein the processor is configured to display to the user on the display a toggle switch for toggling between the first and second ablation modes, and based on the toggling, the processor is configured detect whether the first or second ablation mode has been selected.

10. The system according to claim 1 or claim 2, wherein the processor is configured, based on the selected ablation mode, to provide the user with an indication for at least one of: (i) positioning the catheter at an ablation site, and (ii) placing the catheter against the tissue with a recommended contact force.

## Patentansprüche

1. System (10), umfassend:
eine Schnittstelle (56), die konfiguriert ist, um einen Läsionsparameter zu empfangen, der eine Spezifikation einer Läsion angibt, die in Gewebe eines Organs (12) unter Verwendung mindestens eines ersten und eines zweiten Ablationsmodus gebildet werden soll, die nacheinander auf mehrere Bereiche (40, 60) des Gewebes angewendet werden sollen; und
einen Prozessor (77), der konfiguriert ist, um (i) auszuwählen, für den ersten und den zweiten Ablationsmodus einen ersten beziehungsweise einen zweiten Satz von Parametern, die, wenn sie auf das Gewebe angewendet werden, die Läsion, die den Läsionsparameter aufweist, bilden, (ii) zu detektieren, für einen Bereich, der aus den mehreren Bereichen (40, 60) ausgewählt ist, ob der erste oder der zweite Ablationsmodus ausgewählt wurde, und (iii) zu steuern einen Katheter (22), der mindestens eine Ablationselektrode (26) aufweist, um auf den Bereich Ablationssignale anzuwenden, die auf dem ausgewählten Ablationsmodus basieren und dem ersten oder dem zweiten Ablationsmodus entsprechen.

2. System nach Anspruch 1, wobei der Prozessor konfiguriert ist, um den ersten und den zweiten Satz von Parametern auszuwählen durch: (i) Berechnen des ersten und des zweiten Ablationsparameters oder (ii) Auswählen des ersten und des zweiten Ablationsparameters basierend auf gespeicherten empirischen Daten, die dem ersten und dem zweiten Ablationsmodus entsprechen.

3. System nach Anspruch 1 oder 2, wobei das Organ ein Herz eines Patienten umfasst, und wobei der Prozessor konfiguriert ist zum: (i) Auswählen des ersten Satzes von Parametern durch Auswählen von Impulsfolgen von Pulsfeldablationsenergie (PFA-Energie), und (ii) Auswählen des zweiten Satzes von Parametern durch Auswählen von Hochfrequenzenergie (HF-Energie).

4. System nach Anspruch 3, wobei der Prozessor konfiguriert ist, um den Katheter zu steuern, um die ausgewählten Impulsfolgen der PFA-Energie auf den Bereich anzuwenden, und anschließend (i) zu detektieren, dass der zweite Ablationsmodus zum Anwenden auf einen anschließenden Bereich, der aus den mehreren Bereichen ausgewählt wird, zweiter Ablationssignale ausgewählt wurde, die sich den ersten Ablationssignalen anschließen, und (iii) zu steuern den Katheter, um die ausgewählte HF-Energie auf den anschließenden Bereich anzuwenden.

5. System nach Anspruch 1 oder 2, wobei der Läsionsparameter mindestens einen geometrischen Parameter umfasst, ausgewählt aus einer Liste geometrischer Parameter, bestehend aus: (i) einer Tiefe der Läsion, (ii) einer Breite der Läsion und (iii) einer Kontinuität der Läsion.

6. System nach Anspruch 5, wobei die Schnittstelle konfiguriert ist, um von einem Benutzer den Läsionsparameter zu empfangen, der basierend auf Anweisungen, die die geometrischen Parameter angeben, bestimmt wird, und der Prozessor konfiguriert ist, um den ersten Satz und den zweiten Satz von Parametern basierend auf den Anweisungen, die durch den Benutzer bestimmt werden, auszuwählen.

7. System nach Anspruch 1, wobei der Prozessor konfiguriert ist zum: (i) Auswählen erster Standardparameter zum Auswählen des ersten Satzes und zweiter Standardparameter zum Auswählen des zweiten Satzes, und (ii) Auswählen des ersten Satzes und des zweiten Satzes von Parametern durch Anpassen der ersten und der zweiten Standardparameter basierend auf einer elektroanatomischen Abbildung des Gewebes.

8. System nach Anspruch 1 oder 2, und umfassend eine Anzeige, wobei, als Reaktion auf das Detektieren der Auswahl des ersten oder des zweiten Ablationsmodus, der Prozessor konfiguriert ist, um einem Benutzer den ersten beziehungsweise den zweiten Satz von Parametern anzuzeigen.

9. System nach Anspruch 8, wobei der Prozessor konfiguriert ist, um dem Benutzer auf der Anzeige einen Kippschalter zum Umschalten zwischen dem ersten und dem zweiten Ablationsmodus anzuzeigen, und basierend auf dem Umschalten, der Prozessor konfiguriert ist, um zu detektieren, ob der erste oder der zweite Ablationsmodus ausgewählt wurde.

10. System nach Anspruch 1 oder 2, wobei der Prozessor konfiguriert ist, um, basierend auf dem ausgewählten Ablationsmodus, dem Benutzer eine Angabe für mindestens eines bereitzustellen von: (i) Positionierung des Katheters an einer Ablationsstelle und (ii) Platzieren des Katheters gegen das Gewebe mit einer empfohlenen Kontaktkraft.

## Revendications

1. Système (10), comprenant :
une interface (56), qui est configurée pour recevoir un paramètre de lésion indiquant une spécification d'une lésion prévue pour être formée dans le tissu d'un organe (12) à l'aide d'au moins l'un des premier et second modes d'ablation prévus pour être appliqués séquentiellement à plusieurs sections (40, 60) du tissu ; et
un processeur (77), qui est configuré pour (i) sélectionner, pour les premier et second modes d'ablation, les premier et second ensembles de paramètres, respectivement, qui, lorsqu'ils sont appliqués au tissu, forment la lésion ayant le paramètre de lésion, (ii) détecter, pour une section choisie parmi les sections multiples (40, 60), si le premier ou second mode d'ablation a été sélectionné, et (iii) commander un cathéter (22) ayant au moins une électrode d'ablation (26), afin d'appliquer à la section des signaux d'ablation en fonction du mode d'ablation choisi et correspondant aux premier ou second modes d'ablation.

2. Système selon la revendication 1, dans lequel le processeur est configuré pour choisir les premier et second ensembles de paramètres en : (i) calculant les premier et second paramètres d'ablation, ou (ii) choisissant les premier et second paramètres d'ablation en fonction de données empiriques stockées correspondant aux premier et second modes d'ablation.

3. Système selon la revendication 1 ou la revendication 2, dans lequel l'organe comprend le cœur d'un patient, et dans lequel le processeur est configuré pour : (i) sélectionner le premier ensemble de paramètres en choisissant des trains d'impulsions d'énergie d'ablation par champ pulsé (PFA), et (ii) sélectionner le second ensemble de paramètres en choisissant l'énergie de radiofréquence (RF).

4. Système selon la revendication 3, dans lequel le processeur est configuré pour commander le cathéter afin d'appliquer les trains d'impulsions choisis d'énergie PFA à la section, et ultérieurement, (i) détecter que le second mode d'ablation a été choisi pour appliquer à une section ultérieure choisie parmi les sections multiples, les seconds signaux d'ablation qui sont ultérieurs aux premiers signaux d'ablation, et (iii) commander le cathéter pour appliquer l'énergie **RF** choisie à la section ultérieure.

5. Système selon la revendication 1 ou la revendication 2, dans lequel le paramètre de lésion comprend au moins un paramètre géométrique choisi dans une liste de paramètres géométriques consistant à : (i) la profondeur de la lésion, (ii) la largeur de la lésion et (iii) la continuité de la lésion.

6. Système selon la revendication 5, dans lequel l'interface est configurée pour recevoir d'un utilisateur le paramètre de lésion déterminé en fonction d'instructions indiquant les paramètres géométriques, et le processeur est configuré pour sélectionner les premier et second ensembles de paramètres en fonction des instructions déterminées par l'utilisateur.

7. Système selon la revendication 1, dans lequel le processeur est configuré pour : (i) sélectionner les premiers paramètres par défaut pour sélectionner le premier ensemble, et les seconds paramètres par défaut pour sélectionner le second ensemble, et (ii) sélectionner les premier et second ensembles de paramètres en ajustant les premier et second paramètres par défaut en fonction d'une cartographie électro-anatomique du tissu.

8. Système selon la revendication 1 ou la revendication 2, et comprenant un écran, dans lequel, en réponse à la détection de la sélection du premier ou second mode d'ablation, le processeur est configuré pour afficher à l'utilisateur les premier ou second ensembles de paramètres, respectivement.

9. Système selon la revendication 8, dans lequel le processeur est configuré pour afficher à l'utilisateur sur l'écran un interrupteur à bascule permettant de basculer entre les premier et second modes d'ablation, et en fonction du basculement, le processeur est configuré pour détecter si le premier ou second mode d'ablation a été sélectionné.

10. Système selon la revendication 1 ou la revendication 2, dans lequel le processeur est configuré, en fonction du mode d'ablation choisi, pour fournir à l'utilisateur une indication pour au moins l'un parmi : (i) positionnement du cathéter au niveau d'un site d'ablation, et (ii) placement du cathéter contre le tissu avec une force de contact recommandée.
